# EUROPÄISCHE PATENTSCHRIFT

(11) **EP 4 238 887 B1**
(45) Veröffentlichungstag und Bekanntmachung des Hinweises auf die Patenterteilung: **06.11.2024**
(21) Anmeldenummer: 22160078.6
(22) Anmeldetag: 03.03.2022
(51) Int. Cl.: B65D 47/38, B65B 65/00, B65B 3/00, B65B 3/04, B65B 39/12, B65D 51/00, A61J 1/06, A61J 1/14, A61M 39/10, A61M 39/16

(54) **VERSCHLUSSSYSTEM FÜR EINEN MEDIKAMENTENBEHÄLTER SOWIE MEDIKAMENTENBEHÄLTER MIT EINEM VERSCHLUSSSYSTEM**
CLOSURE SYSTEM FOR A DRUG CONTAINER AND DRUG CONTAINER WITH A CLOSURE SYSTEM
SYSTÈME DE FERMETURE POUR UN RÉCIPIENT À MÉDICAMENTS, AINSI QUE RÉCIPIENT À MÉDICAMENTS DOTÉ D'UN SYSTÈME DE FERMETURE

(43) Veröffentlichungstag der Anmeldung: 06.09.2023
(73) Patentinhaber: Inductio AG, 4500 Solothurn (CH)
(72) Erfinder: Koller, Horst, 8730 Uznach (CH)
(74) Vertreter: Tergau & Walkenhorst Intellectual Property GmbH

(56) Entgegenhaltungen:
- WO-A1-2020/252243
- DE-U1- 9 306 796
- US-A- 4 954 149
- US-A- 5 202 093
- US-A1- 2004 141 886
- US-A1- 2013 270 820
- US-A1- 2018 148 232

## Beschreibung

Die Erfindung betrifft ein Verschlusssystem für einen Medikamentenbehälter, dessen Innenraum über einen in der Art einer Flaschenmündung ausgestalteten Mündungsbereich zugänglich ist, wobei das Verschlusssystem zusätzlich zu einer auf den Mündungsbereich aufschiebbaren, mit einem Dichtelement versehenen Aufprellkappe einen auf diese aufschiebbaren Sicherungsring umfasst, der in einer vollständig auf die Aufprellkappe aufgeschobenen Position mittels einer Anzahl von Schnapprippen an der Aufprellkappe rastbar fixierbar ist. Sie bezieht sich weiter auf einen Medikamentenbehälter mit einem solchen Verschlusssystem sowie auf eine Verwendung des Verschlusssystems.

Medikamente oder Arzneimittel, wie beispielsweise auch Impfstoffe, werden üblicherweise in Wirkstoff- oder Medikamentenbehältern, auch als Container oder Phiole bezeichnet, bereitgestellt. Ein solcher Medikamentenbehälter ist üblicherweise in der Art eines Fläschchens ausgestaltet und umfasst einen Innenraum, in dem das Medikament oder der Wirkstoff vorgehalten wird und der über eine in der Art einer Flaschenmündung ausgestaltete Behälteröffnung zugänglich ist. Von einem solchen Behälter oder Container aus wird der Wirkstoff dann über geeignete Transfersysteme für die eigentliche Verabreichung an geeignete Systeme wie beispielsweise eine Spritze oder eine intravenöse Leitung, die einen Flüssigkeitszugang zum Kreislauf des Patienten be-reitstellt, übergeben.

Die Befüllung derartiger Medikamentenbehälter mit Wirkstoff erfolgt üblicherweise automatisiert in geeignet ausgelegten Befüllanlagen, in denen eine Vielzahl von Medikamentenbehältern gleichzeitig bearbeitet und befüllt werden kann. Dabei werden die Medikamentenbehälter chargenweise mit einer Anzahl von beispielsweise 100 Stück pro Charge zusammengefasst und gemeinsam befüllt (so genanntes "Nesting"). Dementsprechend sollten die Medikamentenbehälter für eine derartige automatisierte Handhabung auch in hohen Stückzahlen geeignet ausgelegt sein; dieser Aspekt ist besonders für Wirkstoffe bedeutsam, die in kurzer Zeit in großen Mengen bereitgestellt werden sollen, wie beispielsweise Impfstoffe.

Aus verschiedenen Gründen, beispielsweise wegen besonders hochwertiger oder ggf auch toxischer oder auf sonstige Weise schädlicher Wirkstoffe, kann es zudem wünschenswert oder sogar notwendig sein, sowohl bei der Befüllung als auch bei der späteren Handhabung, Lagerung und Wirkstoffentnahme unbeabsichtigte Wirkstoffverluste möglichst gering zu halten oder ganz zu vermeiden. Für eine ordnungsgemäße Funktionsweise derartiger Systeme mit der angestrebten Vermeidung unbeabsichtigter Wirkstoffverluste ist kann unter anderem die geeignete Ausgestaltung der Medikamentenbehälter notwendig sein. Üblicherweise sind die Wirkstoff- oder Medikamentenbehälter dazu mit geeigneten Verschlusssystemen versehen, bei denen ein Verschlussstopfen die Behälteröffnung verschließt. Dieser Verschlussstopfen kann dann für eine Entnahme des Medikaments beispielsweise mittels einer Hohlnadel durchstochen werden, über die das Medikament dann aus dem Behälter herausgesaugt werden kann. Zur Fixierung des Verschlussstopfens kann dabei eine Aufprellkappe mit einem eine zentrale Öffnung aufweisenden Ringdeckel vorgesehen sein, der an der "Flaschenmündung" mit der Behälteröffnung angebracht werden kann. Der Verschlussstopfen wird dann zentral in diesem Ringdeckel angebracht.

Ein derartiges System der oben genannten Art, das den genannten Erfordernissen automatisierter Handhabbarkeit einerseits und einer besonders hohen Dichtigkeit andererseits in besonders weitgehendem Umfang genügt, ist beispielsweise aus der nicht vorveröffentlichen Europäischen Patentanmeldung Nummer 20209611, aus Gebrauchsmuster DE9306796U1 oder aus US Patenten US5202093 und US2004141886 bekannt.

Allerdings hat sich herausgestellt, dass zusätzlich zu den genannten Erfordernissen gerade im Hinblick auf die Produktion oder Abfüllung großer Mengen an Wirkstoff auch die Sterilität im Gesamtprozess eine besondere Rolle spielen kann. Üblicherweise werden Medikamentenbehälter bzw. die Komponenten, aus denen sie zusammengesetzt sind, bei oder unmittelbar nach ihrer Herstellung geeignet sterilisiert und sodann steril verpackt. In der sterilen Verpackung werden sie sodann einer weiteren Verwendung, beispielsweise der Befüllung, zugeführt. Die Befüllung selber sollte dann üblicherweise ebenfalls unter sterilen oder aseptischen Bedingungen vorgenommen werden, so dass eine Kontamination oder Verunreinigung des eingefüllten Wirkstoffs sicher ausgeschlossen ist. Eine solche durchgängige Einhaltung der Sterilitätskette kann aber mit erhöhtem Aufwand verbunden sein, gerade wenn in einem Zwischenschritt bei der Handhabung der Medikamentenbehälter die Sterilität nicht eingehalten werden kann und demzufolge eine erneute Sterilisierung des Materials erforderlich wird.

Der Erfindung liegt daher die Aufgabe zugrunde, ein Verschlusssystem der oben genannten Art für einen Medikamentenbehälter anzugeben, mit dem selbst bei der Einhaltung hoher Dichtigkeitskriterien auch die Einhaltung steriler Randbedingungen während der Handhabung des Systems, insbesondere bei der Befüllung, besonders begünstigt ist. Des Weiteren sollen ein Medikamentenbehälter mit einem solchen Ver-schlusssystem sowie eine besonders günstige Verwendung eines solchen Verschlusssys-tems angegeben werden.

Bezüglich des Verschlusssystems wird diese Aufgabe erfindungsgemäß dadurch gelöst, dass der Verschlusskörper des Verschlussstopfens als Entenschnabelventil ausgestaltet ist.

Entenschnabelventile, auch als Entenschnabel-Rückschlagventil bezeichnet, werden unter anderem in Flüssigkeitstransfersystemen verwendet, bei denen abhängig von der Durchflussrichtung des Mediums oder der Flüssigkeit das Ventil öffnet bzw. schließt. Damit kann bei üblichen Anwendungen ein unerwünschter Rückfluss des Mediums unterbunden werden. Zu diesem Zweck umfasst ein solches Entenschnabelventil eine Anzahl von, üblicherweise zwei, aufeinander zulaufenden Ventilflanken, die im "unbelasteten" Ruhezustand entlang einer Kontaklinie aneinander liegen. Falls nun in einem Flüssigkeitskanal die Ventilflanken "in Durchflussrichtung" mit einem Überdruck beaufschlagt werden, werden die Ventilflanken aufgrund der elastischen Eigenschaften des den Ventilkörper bildenden Materials auseinandergedrängt und geben eine Durchflussöffnung für das Medium frei. Ein Durchfluss des Mediums in Öffnungsrichtung ist damit ermöglicht. Falls hingegen eine Beaufschlagung mit Überdruck in Gegenrichtung, also "in Rückflussrichtung", erfolgt, werden die Ventilflanken im Bereich der Kontaktlinie aneinander gepresst und verschließen somit das Ventil; ein Rückfluss ist dann nicht oder zumindest nur sehr geringfügig möglich.

Vorteilhafte Ausgestaltungen der Erfindung sind Gegenstand der Unteransprüche.

Die Erfindung geht von der Überlegung aus, dass für eine besondere Begünstigung der Einhaltung von Sterilitätsbedingungen mögliche Quellen, mit denen ein unerwünschter Eintrag von Verunreinigungen oder Kontaminationen in den Innenraum des Behälters erfolgen könnte, möglichst weitgehend eliminiert werden sollten. Als eine solche mögliche Quelle wird vorliegend insbesondere die Phase angesehen, in der der Behälter mit dem Medikament oder Wirkstoff befüllt wird, da in dieser Phase ein offener Zugang zum Behälterinneren bereitgestellt werden muss. Bei der Befüllung wird in bekannten Systemen üblicherweise eine als Teil des Verschlusssystems vorgesehene Verschlussmembran mittels einer geeigneten Nadel durchstochen, um den erwünschten Zugang zum Behälterinnenraum zu schaffen. Gerade dieses Durchstoßen könnte aber zu einem unerwünschten Eintrag von Verunreinigungen in den Innenraum führen, da an der äußeren Oberfläche der Verschlussmembran anhaftende Partikel oder sonstige Verunreinigungen durch die Nadelspitze in den Innenraum eingetragen werden können.

Daher ist vorliegend vorgesehen, dieses Durchstoßen zu vermeiden; stattdessen ist durch die Ausgetaltung des Verschlusskörpers als Entenschnabelventil erreicht, dass das Nadelsystem beim Einführen in den Behälterinnenraum die Ventilflanken lediglich verdrängt oder verschiebt, eben ohne dass dabei ein Durchstoßen einer Membran oder dergleichen erforderlich wäre. Beim Einbringen des Nadelsystems in den Schnabelbereich des Ventils werden die das Ventil bildenden, aufeinander zu laufenden Flankenflächen auseinandergeschoben, wobei sich das Ventil öffnet. Beim Herausziehen des Nadelsystems nach dem Einfüllen des Medikaments oder Wirkstoffs schließen sich die Flankenflächen dann aufgrund der elastischen Rückstellkräfte im Ventilkörper selbsttätig wieder weitgehend.

Eine besonders hohe Dichtwirkung allgemein ist zudem erreichbar, indem vorteilhafterweise an den Verschlusskörper des Verschlusstopfens radial umlaufend ein Radial-Dichtelement mit einem an die lichte Weite der Behälteröffnung angepassten, im Hinblick auf die Verformbarkeit des Materials des Verschlussstopfens geringfügig größer als die lichte Weite der Behälteröffnung gewählten Querschnitt angeformt ist. Damit kann nämlich die durch den Verschlussstopfen erreichbare Dichtwirkung weiter erhöht werden, indem zusätzlich zu den üblicherweise genutzten axialen auch radiale Kraftkomponenten, also Anpresskräfte, die den Verschlusstopfen in radialer Richtung an die Innenseite der Behältermündung andrücken, nutzbar gemacht werden. Um dem Rechnung zu tragen, ist der Verschlussstopfen in seinem in die Behälteröffnung hineinragenden Bereich hinsichtlich der Formgebung und Dimensionierung seines Querschnitts vorteilhafterweise derart ausgestaltet, dass unter Berücksichtigung der Verformbarkeit seines Materials eine flächige Andrück- oder Anpresswirkung an die Innenwand der Behältermündung entsteht.

Für eine besonders sichere und zuverlässige Handhabung sollte das Verschlusssystem nach seiner Anbringung am Medikamentenbehälter besonders gesichert sein. Dazu umfassend es vorteilhafterweise als weitere Komponente einen auf die Aufprellkappe aufschiebbaren Sicherungsring, der mittels eines innenseitig angeformten, endseitig umlaufend angeordneten Schnapprandes rastend an der Aufprellkappe fixierbar ist. Nach der Anbringung der Aufprellkappe, bei der die an ihrem Außenumfang angeordneten Rastelemente beim Aufschieben auf die Behältermündung mit der an der Behälteröffnung umlaufend angebrachten Außenwulst in Eingriff gebracht, also "aufgeschnappt" oder eingerastet, werden, kann der Sicherungsring auf die Aufprellkappe aufgeschoben werden. Dabei umschließt er die Rastelemente ringförmig von außen und fixiert sie somit in der eingerasteten Stellung; ein Ausweichen nach außen und ein damit verbundenes Lösen der Verrastung ist dann nicht mehr möglich.

Um eine besonders hoher Eignung auch für automatisierte Befüllvorgängezu ermöglichen, ist die oder jede in vollständig auf die Aufprellkappe aufgeschobener Position zur Fixierung des Sicherungsrings vorgesehene Schnapprippe in vorteilhafter Weiterbildung bei einer Bewegung der inneren Mantelfläche des Sicherungsrings relativ zu einer zu dieser korrespondierenden äußeren Mantelfläche der Aufprellkappe in einer korrespondierenden Führungskulisse geführt. Damit ist auch für hohe Takt- oder Durchsatzraten mit den entsprechend hohen Verarbeitungsgeschwindigkeiten bei einer Vormontage der Medikamentenbehälter eine besonders zuverlässige Führung der vormontierten Komponenten relativ zueinander sichergestellt. Damit kann insbesondere erreicht werden, dass potenzielle Störquellen bei der automatisierten Verarbeitung, beispielsweise durch Verkippen oder Verkanten der Komponenten, Fehlpositionierungen oder dergleichen, beseitigt werden. Die erwünschte zuverlässige Führung der vormontierten Komponenten relativ zueinander kann dabei auf besonders einfache Weise erreicht werden, indem unter Rückgriff auf üblicherweise ohnehin vorhandene Komponenten, vorliegend die jeweiligen Schnapprippen, eine Führungspaarung für die Komponenten gebildet wird. Zur Bildung einer solchen Führungspaarung sollte der jeweiligen Schnapprippe im jeweils anderen Bauteil eine korrespondierende Führungskulisse, beispielsweise in der Art einer Nut, zugeordnet werden.

Der als Ventil ausgestaltete Verschlusskörper des Behälterinnenraums könnte eben aufgrund seiner Ventilfunktion möglicherweise an sich nicht den höchsten Dichtigkeitsanforderungen genügen. Um dem Rechnung zu tragen und auch für das vorstehend erläuterte Verschlusssystem die Einhaltung auch höchster Dichtigkeitsanforderungen zu gewährleisten, ist in als eigenständig erfinderisch angesehener Ausgetaltung dem Verschlusskörper des Verschlussstopfens in der Art einer funktionalen Ergänzung ein weiteres Dichtungselement in Form einer durchstechbaren Siegelmembran zugeordnet. Dazu ist an Aufprellkappe ein ihre zentrale Öffnung umlaufender Haltekragen für eine zugeordnete Verschlusskappe angeformt, so dass die Verschlusskappe mittels dieses Haltekragens auf einfache Weise an der Aufprellkappe angebracht werden kann. In weiterer vorteilhafter Ausgestaltung weist Verschlusskappe dabei einen Haltering auf, an dem die im montierten Zustand auf dem Verschlusskörper aufliegende, durchstechbare Siegelmembran angeordnet ist.

Vorteilhafterweise ist das Verschlusssystem mit einem Originalitätsverschluss für den Medikamentenbehälter in der Art eines Einwegverschlusses versehen. Dieser Einwegverschluss, der beispielsweise einen abreissbaren oder verplombt ausgeführten Siegeldeckel umfassen kann, erlaubt eine problemlose und zuverlässige Identifikation, ob der Container bereits zum Flüssigkeitstransfer verwendet wurde oder nicht, und erleichtert somit die Zuordnung, ob der Container bereits "angebrochen" ist und somit bevorzugt für eine weitere Flüssigkeitsentnahme verwendet werden sollte, bis er vollständig entleert ist und somit entsorgt werden sollte. Vorteilhafterweise ist dazu am Haltering der Verschlusskappe zur Bildung eines Originalitätsverschlusses eine vom Haltering abreißbare Siegelplatte angeordnet.

Die Verschlusskappe ist für eine besonders leichte Montierbarkeit und insbesondere auch für eine vorübergehende Entfernung von der Aufprellkappe in der Art eines Bajonettschverschlusses an der Aufprellkappe befestigt. Unter Bajonettverschluss ist hierbei insbesondere zu verstehen, dass die Verschlusskappe mittels einer mehr oder minder weit reichenden Verdrehung an der Aufprellkappe befestigt werden kann. Dazu ist vorteilhafterweise der Haltekragen der Aufprellkappe außenseitig mit einer Anzahl von Führungskulissen versehen, in denen jeweils ein korrespondierender, innenseitig am Haltering der Verschlusskappe angeordneter Riegelzapfen geführt ist.

Ein Medikamentenbehälter, dessen Innenraum über eine in der Art einer Flaschenmündung ausgestaltete Behälteröffnung zugänglich ist, verschlossen mit einem Verschlusssystem der vorstehend beschriebenen Art, wird ebenfalls als eigenständig erfinderisch angesehen.

Ebenfalls als eigenständig erfinderisch angesehen wird die Verwendung eines Verschlusssystems der genannten Art für einen Medikamentenbehälter.

Als eigenständig erfinderisch wird ebenfalls eine Befüllvorrichtung für einen Medikamentenbehälter angesehen, die eine mit einem Vorratsbehälter für eine abzufüllende Substanz verbundenen Injektoreinheit umfasst, die eine in einem äußeren Schutzrohr geführte Füllnadel aufweist.

Die mit der Erfindung erzielten Vorteile bestehen insbesondere darin, dass durch die Ausgestaltung des Verschlusskörpers des Verschlussstopfens als Entenschnabelventil die Befüllung des Medikamentenbehälters durch den Verschlussstopfen hindurch mittels eines geeigneten Füllnadelsystems erfolgen kann, indem das Nadelsystem die Komponenten des Ventils lediglich verdrängt oder verschiebt, ohne dass dabei ein Durchstoßen einer Membran oder dergleichen erforderlich wäre. Beim Einbringen des Nadelsystems in den Schnabelbereich des Ventils werden die das Ventil bildenden, aufeinander zu laufenden Flankenflächen auseinandergeschoben, wobei sich das Ventil öffnet. Beim Herausziehen des Nadelsystems nach dem Einfüllen des Medikaments oder Wirkstoffs schließen sich die Flankenflächen dann aufgrund der elastischen Rückstellkräfte im Ventilkörper selbsttätig wieder weitgehend. Damit ist insgesamt gesehen das Risiko eines Eintrags von Verunreinigungen, äußeren Partikeln oder dergleichen besonders gering gehalten, so dass mit einem solchen Verschlusssystem die Befüllung auch unter hohen Reinheitsanforderungen, insbesondere unter Sterilitätsbedingungen, besonders zuverlässig möglich ist.

Ein Ausführungsbeispiel der Erfindung wird anhand einer Zeichnung näher erläutert. Darin zeigen:
- Fig. 1: einen mit einem Verschlusssystem verschlossenen Medikamentenbehälter,
- Fig. 2: den Medikamentenbehälter nach Fig. 1 mit zugeordnetem Verschlusssystem in perspektivischer Ansicht in Explosionsdarstellung,
- Fig. 3: den Medikamentenbehälter nach Fig. 1 mit zugeordnetem Verschlusssystem im Längsschnitt in Explosionsdarstellung,
- Fig. 4: das Verschlusssystem des Medikamentenbehälters nach Fig. 1 im perspektvischen Schnitt in Explosionsdarstellung,
- Fig. 5: das Verschlusssystem des Medikamentenbehälters nach Fig. 1 im perspektvischen Schnitt in vormontiertem Zustand,
- Fig. 6: einen Verschlussstopfen des Medikamentenbehälters gem. Fig. 1 in unterschiedlichen perspektivischen Ansichten (Fig. 6a, 6b) sowie im Längsschnitt (Fig. 6c),
- Fig. 7: eine alternative Ausführungsform eines Verschlussstopfens des Medikamentenbehälters gem. Fig. 1 in unterschiedlichen perspektivischen Ansichten (Fig. 7a, 7b) sowie im Längsschnitt (Fig. 7c),
- Fig. 8: eine Siegelplatte des Verschlusssystems nach Fig. 1 in perspektivischer Ansicht von unten,
- Fig. 9: die Aufprellkappe des Verschlusssystems des Medikamentenbehälters nach Fig. 1,
- Fig. 10: die Aufprellkappe gem. Fig. 9 mit eingelegtem Verschlusstopfen und aufgeschobenem Sicherungsring,
- Fig. 11: eine Sequenz von Schritten bei der Anbringung des Verschlusssystems an den Medikamentenbehälter nach Fig. 1 zur Vormontage,
- Fig. 12: eine Sequenz von Schritten bei der Befüllung des mit dem Verschlusssystem vormontierten Medikamentenbehälters nach Fig. 1 mit einem Wirkstoff,
- Fig. 13: in vergrößerter Schnittdarstellung eine Sequenz von Schritten bei der Einbringung eines Füllnadelsystems in den Medikamentenbehälter nach Fig. 1,
- Fig. 14: eine Draufsicht auf die Aufprellkappe gem. Fig. 9 mit eingeschobenem Füllnadelsystem,
- Fig. 15: den Mündungsbereich des Entenschnabelventils mit eingeschobener Füllnadel in zwei unterschiedlichen Varianten im Querschnitt, und
- Fig. 16: schematisch ein Ablaufschema für die automatisierte Befüllung einer Vielzahl von Medikamentenbehältern gem. Fig. 1 mit Wirkstoff.

Gleiche Teile sind in allen Figuren mit denselben Bezugszeichen versehen.

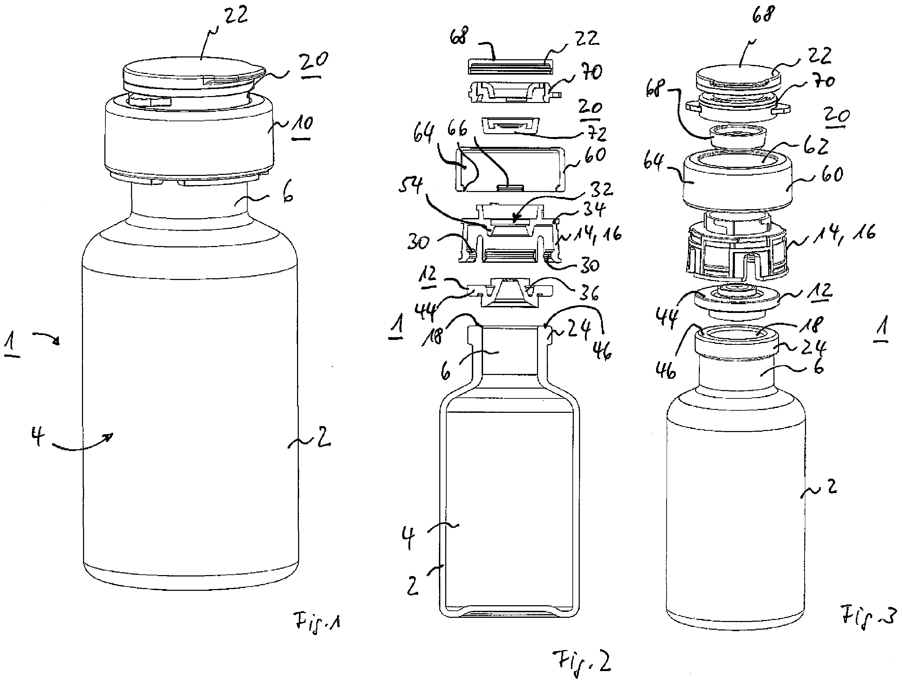

Der Medikamentenbehälter 1 gem. Fig. 1, auch als Container oder Phiole bezeichnet, ist in der Art eines Fläschchens ausgestaltet. Er umfasst einen von einer Behälterwand 2 umschlossenen Innenraum 4, in dem das Medikament oder der Wirkstoff vorgehalten wird. Die Behälterwand 2 ist im Ausführungsbeispiel aus einem geeignet gewählten Kunststoff mit oder ohne Barriereschicht gefertigt. Besonders bevorzugt ist hierbei ein "medical grade" Kunststoff vorgesehen wie beispielsweise COP Varianten 690R^{®}, 790R^{®}, COC Varianten Topas^{®} 8007S-04, 6013S-04, 6015S-04. Ganz besonders bevorzugt ist der Kunststoff dabei im Hinblick auf die Kriterien transparent, bruchunempfindlich, geringe bis keine Wechselwirkung mit dem vorgesehenen Medikament, medical grade, insbesondere verwendbar als Glasersatz, einzeln oder in Kombination miteinander, geeignet ausgewählt. Der Innenraum 4 ist über eine in der Art einer Flaschenmündung ausgestaltete Behälteröffnung 6 zugänglich.

Der Medikamentenbehälter 1 ist in besonderem Maße für eine automatisierte Befüllung mit Wirkstoff unter aseptischen oder sterilen Bedingungen geeignet ausgelegt. Dabei sollen insbesondere die Komponenten des Medikamentenbehälters nach ihrer Herstellung vollständig oder bedarfsgerecht sterilisiert und anschließend unter sterilen Bedingungen weiterbehandelt, gelagert oder auf sonstige Weise einer Benutzung zugeführt werden, ohne dass nachträglich eine erneute vollständige Sterilisierung erforderlich werden soll. Als Schwachpunkt wird diesbezüglich vorliegend insbesondere die Befüllung des Behälters mit Wirkstoff angesehen, da hierzu der Behälterinnenraum 4 zugänglich gemacht werden muss und somit ein Eintrag von Verunreinigungen oder Kontaminationen denkbar wäre. Um den damit im Hinblick auf die gewünschte durchgängige Aufrechterhaltung der Sterilität einhergehenden Anforderungen auf besonders weitgehende Weise gerecht zu werden, ist der Medikamentenbehälter 1 mit einem die Behälteröffnung 6 verschließenden Verschlusssystem 10 ausgerüstet, mit dem einerseits gerade bei der Befüllung das Risiko eines Eintrags unerwünschter Verunreinigungen besonders gering gehalten werden kann, wobei andererseits auch ein besonders wirksamer Manipulationsschutz gegeben sein soll.

Das Verschlusssystem 10 umfasst, wie dies der Darstellung im Längsschnitt in Fig. 2 sowie der perspektivischen Ansicht in Fig. 3, jeweils in Explosionsdarstellung, deutlich entnehmbar ist, als wesentliche Elemente einen Verschlussstopfen 12, mit dem die Behälteröffnung 6 des Behälterinnenraums 4 verschlossen ist, eine als Aufprellkappe 14 ausgestaltete Fixierkappe 16, mit der der Verschlussstopfen 12 auf der Mündungsöffnung 18 des Medikamentenbehällters 1 befestigt ist, sowie eine Verschlusskappe 20 mit daran angebrachtem Originalitätsverschluss 22. Deren bevorzugte Ausgestaltungen und jeweilige Funktionen werden nachfolgend näher beschrieben.

Die Behälterwand 2 des Medikamentenbehälters 1 ist im Bereich der Behälteröffnung 6 mit einer Anzahl von, im Ausführungsbeispiel einer, umlaufend angebrachten Außenwulst 24 als Befestigungselement für das Verschlusssystem 10 versehen. Im Ausführungsbeispiel ist, wie dies aus der Darstellung in Fig. 2 gut ersichtlich ist, die Außenwulst 24 vollständig umlaufend ausgeführt und in der Nähe der Mündungsöffnung 18 der Behälteröffnung 6 positioniert. In anderen Ausführungsformen könnte die Außenwulst 24 ganz oder teilweise segmentiert ausgeführt sein, d. h. mehrere in rotatorischer Richtung gesehen aufeinander folgende, beabstandet zueinander positionierte Segmente unter Bildung von dazwischen befindlichen Lücken umfassen.

Wesentliche Komponente des Verschlusssystems 10 sind das als Verschlusstopfen 12 ausgeführte Dichtelement zum Verschließen der Behälteröffnung 6 und die Fixierkappe 16, mit der der Verschlussstopfen fest an der Behälteröffnung 6 angebracht werden kann. Angepasst an die Außenwulst 24 ist die Fixierkappe 16 als Aufprellkappe 14 ausgeführt, an deren Außenumfang eine Anzahl von mit der jeweiligen Außenwulst 24 in Eingriff bringbaren Schnapphaken oder Rastelementen 30 angeordnet ist. Beim Anbringen der Aufprellkappe 14 kann diese somit auf die Behälteröffnung 6 aufgesteckt oder aufgeprellt werden, wobei die Rastelemente 30 zunächst durch die jeweilige Außenwulst 24 nach außen gebogen werden und anschließend, nach weiterem Aufschieben, die Außenwulst 24 hintergreifen und in der Art einer Schnappverbindung mit dieser verrasten.

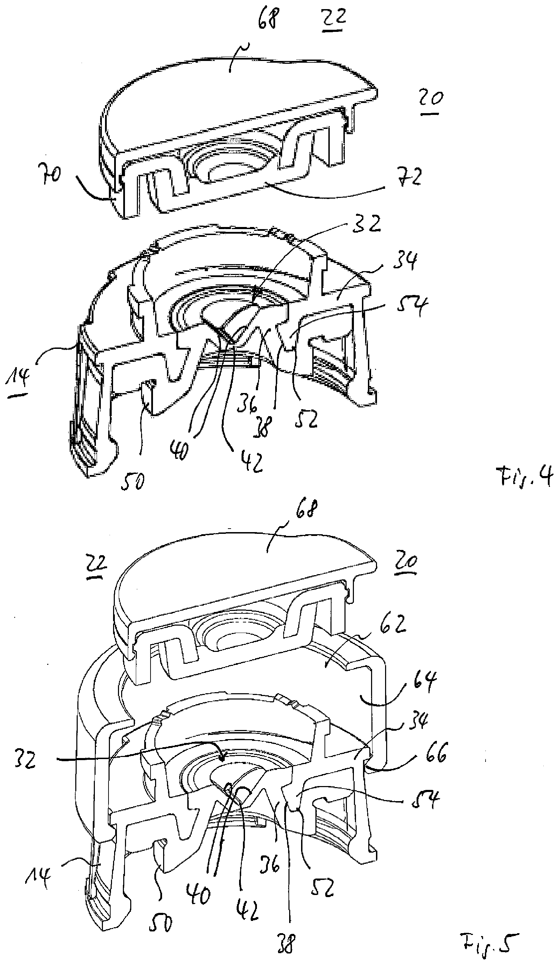

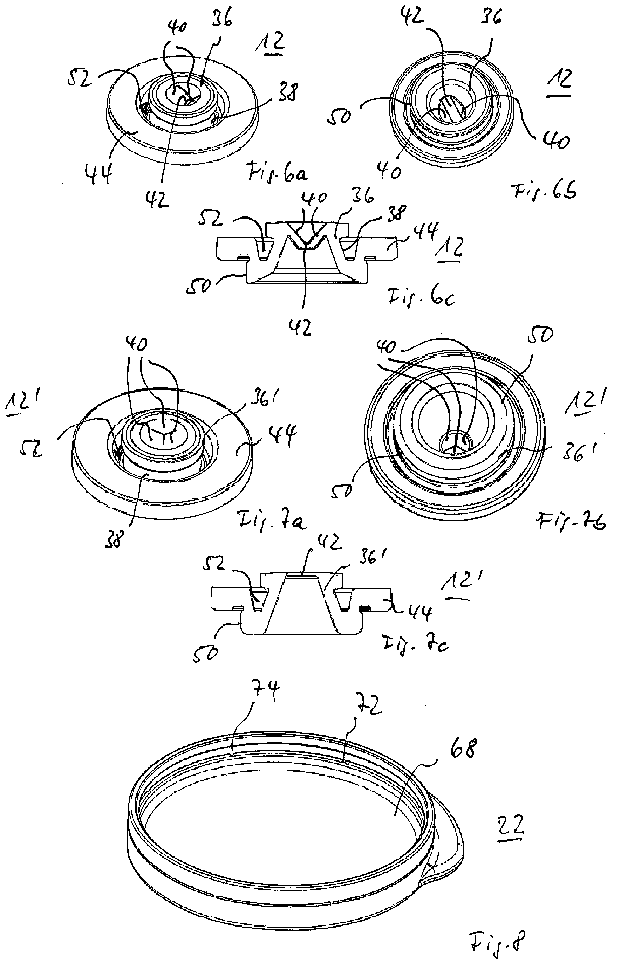

Die Aufprellkappe 14 umfasst einen eine zentrale Öffnung 32 aufweisenden Ringdeckel 34. Der an sich einstückig ausgeführte, in den perspektivischen Schnitten in Fig. 4 und 5 im montierten Zustand und in Fig. 6 in verschiedenen Ansichten vergrößert gezeigte Verschlussstopfen 12 umfasst hingegen in der Art eines Grundelements einen zentralen Verschlusskörper 36, der an seinem "oberen", im montierten Zustand vom Behälterinnenraum 4 abgewandten Ende in der Öffnung 32 mit dem Ringdeckel 34 rastend in Eingriff bringbar ist. Dazu ist der Verschlusskörper 36 in seinem Verbindungsbereich mit dem Ringdeckel 34 mit einer eine Hinterschneidung bildenden umlaufenden Nut 38 versehen. Der an sich einstückige Verschlussstopfen 12 ist, auch im Hinblick auf die erwünschten Dichtzwecke, aus einem geeigneten und zudem vergleichsweise weichen und gut verformbaren Material, im Ausführungsbeispiel aus Gummi oder aus TPE, bevorzugt "medical grade", gefertigt. Unter Nutzung dieser Materialeigenschaften, insbesondere der Verformbarkeit, kann der Verschlussstopfen 12 mit der Aufprellkappe 14 annähernd fest verbunden werden, indem der Verschlusskörper 36 in die Öffnung 32 im Ringdeckel 34 eingebracht wird und der umlaufende Rand der Öffnung 32 anschließend in die Nut 38 eingreift und damit das Dichtelement 12 an der Aufprellkappe 14 fixiert.

Der Verschlussstopfen 12 ist gezielt dafür ausgelegt, bei einem Medientransfer in den Medikamentenbehälter 1 hinein, also bei der Befüllung, und auch aus dem Medikamentenbehälter heraus, also bei Entnahme von Wirkstoff aus dem Behälterinnenraum 4, das Risiko eines Eintrags von Verunreinigungen, äußeren Partikeln oder dergleichen besonders gering zu halten. Damit soll insbesondere erreicht werden, dass die Befüllung des Behälterinnenraums 4 und auch eine Entnahme von Wirkstoff auch unter hohen Reinheitsanforderungen, insbesondere unter Sterilitätsbedingungen, sichergestellt werden kann. Um dies zu ermöglichen, ist der Verschlusskörper 36 des Verschlussstopfens 12 als so genanntes Entenschnabelventil ausgestaltet.

Ein solches Entenschnabelventil, auch als Entenschnabel-Rückschlagventil bezeichnet, wird weit verbreitet in Flüssigkeitstransfersystemen verwendet, bei denen abhängig von der Durchflussrichtung des Mediums oder der Flüssigkeit das Ventil öffnet bzw. schließt. Damit kann bei üblichen Anwendungen ein unerwünschter Rückfluss des Mediums unterbunden werden. Zu diesem Zweck umfasst ein solches Entenschnabelventil eine Anzahl von, üblicherweise zwei, aufeinander zulaufenden Ventilflanken 40, die im "unbelasteten" Ruhezustand entlang einer Kontaklinie 42 aneinander liegen. Falls nun in einem Flüssigkeitskanal die Ventilflanken "in Durchflussrichtung" mit einem Überdruck beaufschlagt werden, werden die Ventilflanken 40 aufgrund der elastischen Eigenschaften des den Ventilkörper bildenden Materials auseinandergedrängt und geben eine Durchflussöffnung für das Medium frei. Ein Durchfluss des Mediums in Öffnungsrichtung ist damit ermöglicht. Falls hingegen eine Beaufschlagung mit Überdruck in Gegenrichtung, also "in Rückflussrichtung", erfolgt, werden die Ventilflanken 40 im Bereich der Kontaktlinie 42 aneinander gepresst und verschließen somit das Ventil; ein Rückfluss ist dann nicht oder zumindest nur sehr geringfügig möglich.

Die Verwendung eines solchen Entenschnabelventils im Verschlusskörper 36 des Verschlusstopfens 12 bewirkt nunmehr erfindungsgemäß, dass ein Medientransfer, also die Befüllung des Medikamentenbehälters 1 oder die Entnahme von Wirkstoff aus dem Medikamentenbehälter 1, durch den Verschlussstopfen 12 hindurch mittels eines jeweils geeigneten Nadelsystems, beispielsweise eine Injektionsnadel oder ein Spikesystem zur Entnahme, erfolgen kann, indem das Füllnadelsystem die Ventilflanken 40 des Ventils lediglich verdrängen oder verschieben muss, ohne dass dabei ein Durchstoßen einer Membran oder dergleichen erforderlich wäre. Beim Einbringen des Füllnadelsystems in den Schnabelbereich des Ventils werden nämlich die das eigentliche Ventil bildenden, aufeinander zu laufenden Ventilflanken 40 auseinandergeschoben, wobei sich das Ventil öffnet. Beim Herausziehen des Füllnadelsystems nach dem Einfüllen des Medikaments oder Wirkstoffs oder dessen Entnahme durch eine Injektionsnadel schließen sich die Ventilflanken 40 dann aufgrund der elastischen Rückstellkräfte im Ventilkörper selbsttätig wieder weitgehend.

In den Darstellungen gem. Fig. 6 ist der Verschlussstopfen 12 in einer Ausgestaltung mit einem Entenschnabelventil in "üblicher" Bauweise, nämlich mit zwei Ventilflanken 40, gezeigt. Alternativ kann das Entenschnabelventil aber auch mit mehr als zwei Ventilflanken 40 ausgeführt sein, die entsprechend dann im Ruhezustand unter Bildung mehrerer Kontaktlinien 42 aneinanderstoßen. Beispielhaft ist hierzu in Fig. 7 ein alternativ ausgeführter Verschlussstopfen 12' gezeigt, dessen Verschlusskörper 36' als Entenschnabelventil mit drei Ventilflanken 40 ausgeführt ist, die an drei Kontaktlinien 42 aneinander anliegen.

In vorteilhafter Weise trägt als Dichtelement ausgeführte Verschlussstopfen 12 auf zweierlei Weise zur Abdichtung der Behälteröffnung 6 bei. Einerseits weist der Verschlusstopfen 12 eine an den Verschlusskörper 36, 36' angeformte, radial um diesen umlaufende Dichtplatte 44 auf. Durch diese wird, mit bekannten System durchaus vergleichbar, eine Dichtwirkung erzielt, indem im montierten System die in ihren Abmessungen, insbesondere ihrem Außendurchmesser, geeignet an den Mündungsrand 46 der Behälteröffnung 6 angepasste Dichtplatte 44 durch die auf den Mündungsrand 46 aufrastbare Aufprellkappe 14 auf den Mündungsrand 46 aufgedrückt wird. Durch diese in Bezug auf die Längsachse der Behälteröffnung gesehen axiale Kraftwirkung kann die Dichtplatte 44 infolge der Verformbarkeit des Materials bereits eine Dichtwirkung entfalten. Darüber hinaus ist vorliegend für eine insgesamt besonders erhöhte Dichtwirkung aber auch noch die Bereitstellung von radialen Kraftkomponenten, also Anpresskräfte, die das Dichtelement in radialer Richtung an die Innenseite der Behälterwand 2 im Bereich ihrer Mündung andrücken, vorgesehen.

Dazu ist "unterhalb" der Dichtplatte 44, also auf ihrer im montierten Zustand dem Behälterinnenraum 4 zugewandten Seite, am Verschlusskörper 36 ein Radial-Dichtelement 50 angeformt. Diese ist in seiner Querschnittsform an die Querschnittsform der Behälteröffnung 6 im Mündungsbereich angepasst (im Ausführungsbeispiel sind beide rund). Hinsichtlich seiner Dimensionierung ist es zudem an die lichte Weite I der Behälteröffnung 6 angepasst und im Hinblick auf die Verformbarkeit des Materials des Dichtelements 12 geringfügig größer als die lichte Weite I der Behälteröffnung 6 ausgeführt. Damit entsteht bei in die Behälteröffnung 6 eingebrachtem Radial-Dichtelement 50 unter Berücksichtigung der Verformbarkeit seines Materials eine flächige Andrück- oder Anpresswirkung an die Innenwand des Behälters im Bereich der Behälteröffnung. Im Hinblick auf gängige Standards und übliche Normen für derartige Komponenten kann die Behälteröffnung geeignet gewählt und dimensioniert sein; beispielsweise kann ihre lichte Weite geeignet abgestimmt auf das Standard-Maß "13er Neck" (entspricht einem Außendurchmesser der Behälteröffnung von 13mm), auf das Standard-Maß "20er Neck" (entspricht einem Außendurchmesser der Behälteröffnung von 20mm) oder abgestimmt auf Sondervarianten für die Neck-Geometrie für den Innendurchmesser gewählt sein.

Der Dichtelement 12 ist dabei vorteilhafterweise für eine noch weiter verbesserte Dichtwirkung in radialer Richtung ausgeführt. Dazu ist die Formgebung derart gewählt, dass der den Verschlusskörper 36, 36' ausbildende Zentralbereich des Verschlussstopfens 12,12' von einer tief in die Dichtplatte 44 hineinreichenden, umlaufenden nut- oder grabenartigen Vertiefung 52 umgeben ist. Die Vertiefung 52 kann dabei auch vollständig die Materialdicke der Dichtplatte 44 durchdringen, so dass das Dichtelement 12,12' in dieser Ausführung mehrkomponentig ausgestaltet ist. Daran angepasst weist die Aufprellkappe 14, wie in den Fig. 4 und 5 erkennbar ist, einen an den Ringdeckel 34 an seiner Unterseite angeformten, die Öffnung 32 umlaufenden Verstärkungsring 54 auf. Bei der Montage der beiden Komponenten wird dieser Verstärkungsring 54 in die Vertiefung 52 des Dichtelements 12 eingebracht. Die Abmessungen sind dabei derart aufeinander abgestimmt, dass der Verstärkungsring 54 dem Radial-Dichtelement 50 des Dichtelements 12 erhöhte Festigkeit und Steifigkeit nach außen hin, also in radialer Richtung, verleiht und damit die radiale Abdichtung noch weiter verbessert. Insbesondere kann durch die entsprechend gewählten Abmessungen der Verstärkungsring 54 das Radial-Dichtelement 50 mehr oder weniger nach außen hin leicht verformen und dabei eine zusätzliche Anpresskraft in radialer Richtung an die Innenwand des Medikamentenbehälters 1 im Bereich der Behälteröffnung 6 erzeugen.

Die Aufprellkappe 14 besteht im Ausführungsbeispiel aus einem geeignet gewählten Kunststoff, nämlich aus Polypropylen (PP), einem Polyolefin, Cyclo-Olefin-Copolymer (COC), Cyclo-Olefine-Polymer (COP) oder Polycarbonat.

Als weitere Komponente, wie dies wiederum aus den Darstellungen in den Fig. 2, 3 und 5 deutlich wird, umfasst das Verschlusssystem 10 einen auf die Aufprellkappe 14 aufschiebbaren Sicherungsring 60. Dieser ist unter Freilassung einer ausreichend groß bemessenen zentralen Öffnung 62 als Ringstruktur mit zylindrischer Mantelfläche 64 ausgestaltet; diese kann nach dem Aufprellen der Aufprellkappe 14 und der Verrastung mit den Außenwulsten 24 von außen umgreifend auf die Aufprellkappe 14 geschoben werden. Damit fixiert der Sicherungsring 60 über seine zylindrische Mantelfläche 64 die Rastelemente 30 radial, so dass diese nicht mehr nach außen ausweichen können. Damit ist die Verrastung der Aufprellkappe 14 mit der Außenwulst 24 nicht mehr ohne weiteres lösbar und somit festgelegt. Der Sicherungsring 60 weist seinerseits eine Anzahl von innenseitig an seiner zylindrischen Mantelfläche angeformten, endseitig positionierten Schnapprippen 66 auf, mittels derer er rastend an der Aufprellkappe 14 fixierbar ist.

Des Weiteren weist der mit dem Verschlusssystem 10 verschlossene Medikamentenbehälter 1 als Komponente den Originalitätsverschluss 22 auf. Dieser soll in der Art eines Einwegverschlusses sicherstellen, dass für den Verwender auf einfache und zuverlässige festgestellt werden kann, ob der Medikamentenbehälter 1 bereits zum Flüssigkeitstransfer verwendet wurde oder nicht, d. h. ob bereits Wirkstoff entnommen wurde oder nicht. Er erleichtert somit die Zuordnung, ob der Container bereits "angebrochen" ist und somit bevorzugt für eine weitere Flüssigkeitsentnahme verwendet werden sollte, bis er vollständig entleert ist und somit entsorgt werden sollte. Der Originalitätsverschluss 22 ist als an die Verschlusskappe 20 angeformte Siegelplatte 68 ausgestaltet, wie dies im perspektivischen Schnitt gem. Fig. 5 erkennbar ist.

Die Verschlusskappe 20 ist dabei in als eigenständig erfinderisch angesehener Ausgestaltung als funktionale Ergänzung zu dem als Entenschnabelventil ausgestalteten Verschlusskörper 36,36' des Verschlussstopfens 12,12' vorgesehen. Da nämlich der Verschlussstopfen 12,12' eben gerade wegen seiner Ausgestaltung als Ventilfunktion möglicherweise nicht vollständig hermetisch dichtend wirken könnte, gerade bei längerer Lagerszeit des befüllten Medikamentenbehälters 1, ist die Verschlusskappe 20 als ergänzendes Dichtmittel ausgestaltet. Dazu umfasst sie eine an einem Haltering 70 angebrachte Siegelmembran 72, die im montierten Zustand des Systems unmittelbar auf dem Verschlusskörper 36,36' aufliegt und somit diesen nach außen hin abdichtet. Die Siegelmembran 72 ist dabei derart dimensioniert und positioniert, dass sie im montierten Zustand die zentrale Öffnung 32 des Ringdeckels 34 und damit die hierüber zugängliche frei liegende Fläche des Dichtelements 12 vollständig überdeckt. Zur Bildung des Originalitätsverschlusses 22 ist am Haltering 70 zudem von diesem abreißbar die Siegelplatte 68 angebracht. Für einen Zugriff auf das Innere des Medikamentenbehälters 1, also für eine Entnahme von Wirkstoff, muss somit zunächst die Siegelplatte 68 entfernt und anschließend die Siegelmembran 72 durchstochen werden, bevor ein entsprechendes Nadelsystem durch das darunterliegende Entenschnabelventil hindurchgeführt werden kann. Zur Verdeutlichung ist in Fig. 8 die Siegelplatte 68 in perspektivischer Ansicht von unten gezeigt, wobei auch einige der Fixierpunkte 74 erkennbar sind, an denen die Siegelplatte 68 abreißbar am Haltering 70 befestigt ist.

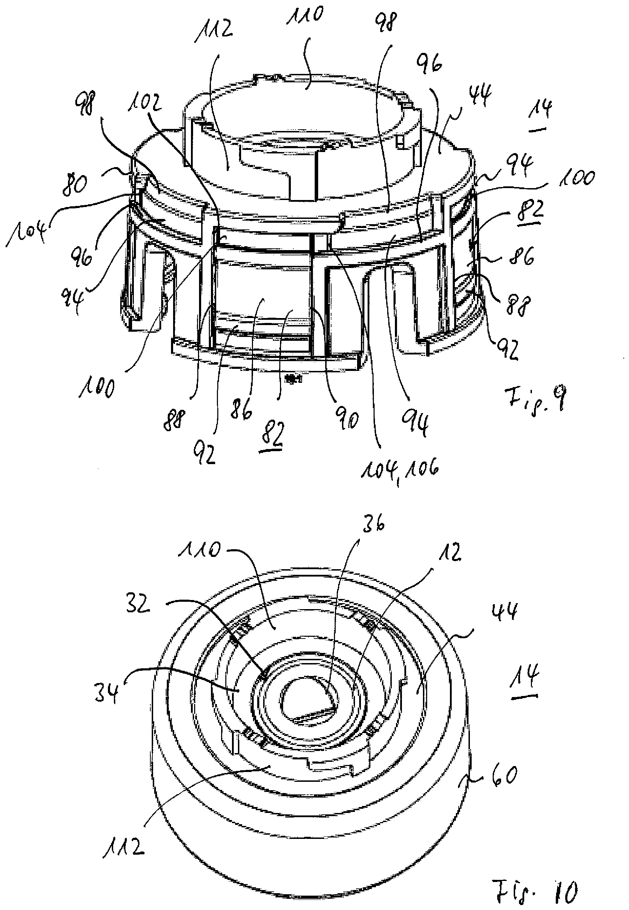

Das Verschlusssystem 10 des Medikamentenbehälters 1 ist in als eigenständig erfinderisch angesehener Ausgetaltung für eine besonders stabile Vormontage des Sicherungsrings 60 an der Aufprellkappe 14 ausgelegt, so dass das vormontierte System auch für nachfolgende Prozessschritte mit hoher Beanspruchung, beispielsweise im Rahmen automatisierter Befüll- oder Verpackungsvorgänge, besonders gut geeignet ist. Dazu werden die am Sicherungsring 60 innenseitig angeordneten Schnapprippen 66 in der Art einer Zusatzfunktion auch zur Bildung einer Führungspaarung herangezogen, die ergänzend dazu, wie in der perspektivischen Ansicht der Aufprellkappe 14 in Fig. 9 gezeigt, für jede der Schnapprippen 66 noch eine in der äußeren Mantelfläche 80 angeordnete Führungskulisse 82 umfasst. Die durch die Schnapprippe 66 einerseits und die korrespondierende Führungskulisse 82 andererseits jeweils gebildete Führungspaarung bewirkt, dass die jeweilige Schnapprippe 66 bei einer Bewegung der inneren Mantelfläche 84 des Sicherungsrings 60 relativ zu der zu dieser korrespondierenden äußeren Mantelfläche 80 der Aufprellkappe 14 in der korrespondierenden Führungskulisse 82 geführt ist, so dass die Positionen dieser Komponenten relativ zueinander reproduzierbar und kontrollierbar eingestellt werden können.

Im Ausführungsbeispiel ist die jeweilige Schnapprippe 66 innenseitig am Sicherungsring 60 und korrespondierend dazu die jeweilige Führungskulisse 82 außenseitig an der Aufprellkappe 14 angeordnet; alternativ könnte aber auch die Schnapprippe 66 an der Aufprellkappe 14 und korrespondierend dazu die Führungskulisse 82 an der inneren Mantelfläche 84 des Sicherungsrings 60 positioniert sein.

Wie der Darstellung in Fig. 9 deutlich entnehmbar ist, umfasst die Führungskulisse 82 ein in der Art einer axialen Nut ausgeführtes, sich in einer axialen Richtung parallel zur Rotationsachse der Aufprellkappe 14 erstreckendes erstes Axialsegment 86. Dieses Axialsegment 86 ist beidseitig durch jeweils eine lineare Führungskante 88, 90 begrenzt, die beim Aufschieben des Sicherungsrings 60 auf die Aufprellkappe 14 die jeweilige Schnapprippe 66 führen. Des Weiteren ist im ersten Axialsegment 86 eine Rastwulst 92 angeordnet. Sobald die Schnapprippe 66 beim Aufschieben des Sicherungsrings 60 auf die Aufprellkappe 14 über die Rastwulst 92 hinweg geschoben wurde, erfolgt die Verrastung der jeweiligen Schnapprippe 66 mit der Rastwulst 92.

Des Weiteren weist die Führungskulisse 82 ein in der Art einer tangentialen Nut ausgeführtes, sich in einer tangentialen Richtung um die Rotationsachse der Aufprellkappe 14 erstreckendes Tangentialsegment 94 auf. Das Tangentialsegment 94 weist in einem ersten Bereich eine untere oder proximale Führungskante 96 auf, oberhalb derer ein Ansatz eines zweiten Axialsegments 98 mit offen gehaltenem Einmündungsbereich ausgebildet ist, in das die jeweilige Schnapprippe 66 eingeschoben werden kann. Die Führungskante 96 bildet beim Aufstecken des Sicherungsrings 60 auf die Aufprellkappe 14 einen Anschlag für die jeweilige Schnapprippe 66 und verhindert somit eine weitere lineare Aufschiebe-Bewegung. Das Tangentialsegment 94 geht im Übrigen in das Axialsegment 86 über, wobei im Übergangsbereich 100 zwischen Tangentialsegment 94 und Axialsegment 86 ein Anschlag 102 für die jeweilige Schnapprippe 66 ausgebildet ist. Dieser dient zur Begrenzung einer Rotation des Sicherungsrings 60 relativ zur Aufprellkappe 14.

Im Tangentialsegment 94 der Führungskulisse 82 ist zudem ein Rastzahn 104 mit angeschrägter Anschlagfläche 106 für die Schnapprippe 66 angeordnet.

Wie zudem der Darstellung der Aufprellkappe 14 in Fig. 9 sowie der Darstellung der Aufprellkappe 14 mit eingebrachtem Verschlussstopfen 12 und aufgeschobenem Siicherungsring 60 in perspektivischer Draufsicht in Fig. 10 gut entnehmbar ist, ist an der Aufprellkappe 14 ein die zentrale Öffnung 32 im Ringdeckel 34 umlaufender Haltekragen 110 angeformt. Dieser ist dafür vorgesehen und ausgestaltet, dass die Verschlusskappe 20 über deren Haltering 70 daran befestigt werden kann. Um dies zu ermöglichen, ist - in gewisser Weise analog zur vorgesehenen Verbindung des Sicherungsrings 60 mit der Aufprellkappe 14 - ebenfalls eine Art Bajonettverschluss vorgesehen, bei dem innenseitig am Haltering 70 angeordnete Nocken in eine zugeordnete, außenseitig am Haltekragen 110 angeformte Führungskulisse 112 eingreifen.

Der Medikamentenbehälter mitsamt seinem Verschlusssystem ist dafür ausgelegt, nach der Produktion der Einzelteile und deren Sterilisierung eine Vormontage derart vorzunehmen, dass das komplette System einem automatisierten Befüllungsprozess zugeführt werden kann. In der ersten Phase erfolgt dabei, nach der Herstellung und Sterilisierung der Komponenten, die Vormontage, bei der das Verschlusssystem 10 mit allen seinen Komponenten gebrauchsfertig vormontiert wird. Anschließend kann es dann an eine geeignete Befülleinrichtung geliefert und dort mit dem Wirkstoff automatisiert befüllt werden.

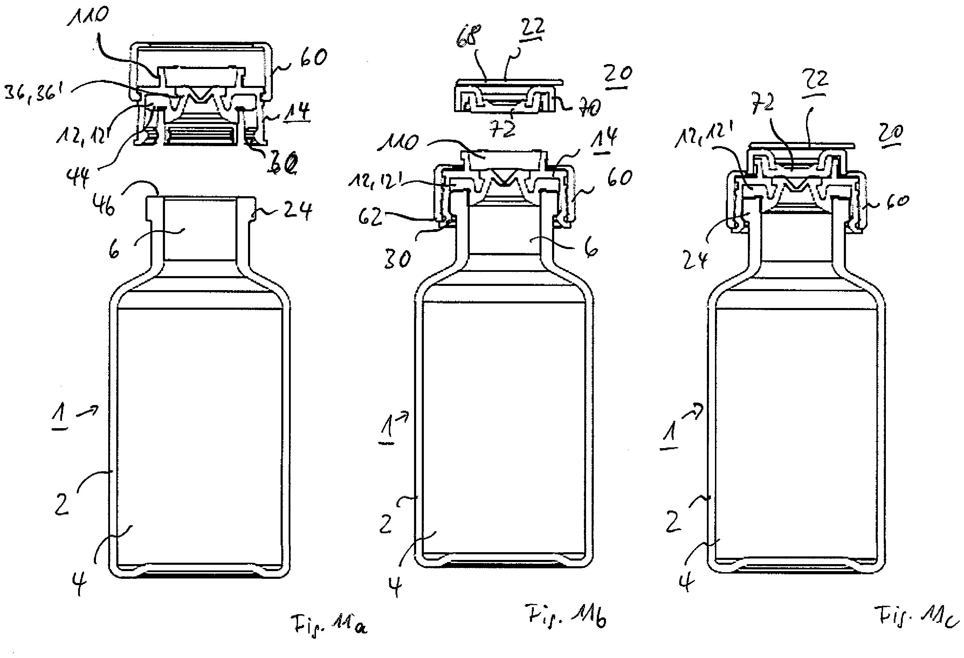

Die Anbringung des Verschlusssystems 10 an den Medikamentenbehälter 1 im Sinne besagter Vormontage ist in Fig. 11 anhand einer Sequenz von Schritten gezeigt. In einem ersten Schritt, in Fig. 11a gezeigt, wird zunächst der versehene Sicherungsring 60 mit seinen Schnapprippen 66 oberhalb der Einmündungsbereiche der jeweiligen zweiten Axialsegmente 98 an der vormontierten, bereits mit dem den Verschlusskörper 36,36' umfassenden Dichtelement 12,12' versehenen Aufprellkappe 14 positioniert. Anschließend wird der Sicherungsring 60 linear nach unten auf die Aufprellkappe 14 gedrückt und damit auf diese aufgesteckt. Dabei tauchen die Schnapprippen 66 in das zweite Axialsegment 98 der jeweils zugeordneten Führungskulisse 82 ein, bis sie an die Führungskante 96 anschlagen. Damit ist bei der Vormontage die jeweilige Schnapprippe 66 zunächst in das Tangentialsegment 94 der ihr zugeordneten Führungskulisse 82 eingeschoben.

Anschließend ist vorgesehen, den Sicherungsring 60 relativ zur Aufprellkappe 14 zu verdrehen. Durch dieses Verdrehen wird die jeweilige Schnapprippe 66 im jeweiligen Tangentialsegment 94 geführt, bis sie an den Anschlag 102 anschlägt. Damit wird die Verdrehung beendet. Bei der Verdrehung wird die Schnapprippe 66 auch über den im Tangentialsegment 94 angeordneten Rastzahn 104 hinwegbewegt, was aufgrund der Abschrägung der Anschlagsfläche 106 in dieser Verdrehrichtung möglich ist. Aufgrund der asymmetrischen Kontur des Rastzahns 104 ist ein Rückwärtsdrehen dann aber nicht mehr möglich, so dass der Sicherungsring 60 gegenüber der Aufprellkappe 14 dann auch rotationsgesichert ist. Der Sicherungsring 60 ist damit sowohl axial als auch rotatorisch in einer definierten und reproduzierbaren Position auf der Aufprellkappe 14 fixiert.

In dieser Position befindet sich die Schnapprippe 66 dann auch im Übergangsbereich 100 vom Tangentialsegment 94 der Führungskulisse 82 zu deren erstem Axialsegment 86. Von dieser Position aus kann somit später das weitere Aufschieben des Sicherungsrings 60 auf die Aufprellkappe 14 in axialer Richtung erfolgen. Die Komponenten befinden sich dabei in sicherem Eingriff miteinander, so dass das solchermaßen vormontierte Verschlusssystem 10 besonders gut auch für eine automatisierte Weiterverarbeitung selbst bei hoher Beanspruchung und hohen Stückzahlen geeignet ist. Dies entspricht dem in Fig. 11a gezeigten Zustand.

Die Aufprellkappe 14 wird dann auf den Mündungsbereich des Medikamentenbehälters 1 aufgesetzt und auf diesen aufgedrückt, bis die Rastelemente 30 die Außenwulst hintergreifen und an dieser einrasten. Dabei dringt das der Verschlusstopfen 12,12' mit seinem Radial-Dichtelement 50 in die Behälteröffnung 6 ein, bis die Dichtplatte 44 mit ihrem äu-βeren Rand auf der Mündung 18 des Behälters 1 aufliegt und anschließend, unter geringfügiger Verformung der Dichtplatte 44 in Längsrichtung der Behälteröffnung 6 gesehen, die Rasthaken 30 der Aufprellkappe 14 unterhalb der Außenwulst 24 einrasten. Anschließend wird dann der Sicherungsring 60 nach unten hin verschoben, so dass seine zylindrische Mantelfläche die Aufprellkappe 14 außenseitig umgreift. Damit werden die Rastelemente 30 in ihrer Position verriegelt, und der Medikamentenbehälter 1 in der in Fig. 11b gezeigten Position ist sicher mit der Aufprellkappe 14 und dem in ihr befindlichen Verschlussstopfen 12,12' verbunden, wobei der an der Aufprellkappe 14 angeformte Haltering 110 "nach oben hin" aus dem Sicherungsring 60 herausragt und somit frei zugänglich ist. In dieser in Fig. 11b gezeigten Position ist der Medikamentenbehälter 1 nunmehr für die vorübergehende Anbringung der Verschlusskappe 20 mitsamt Originalitätsverschluss 22 und Siegelmembran 72 am Haltering 110 bereit.

Eine solche vorübergehende Anbringung kann in als eigenständig erfinderisch angesehener Ausgestaltung insofern bedeutsam sein, als dass die Verschlusskappe 20 nach erfolgter Anlieferung des vormontierten Medikamentenbehälters 1 an eine Befüllanlage zunächst wieder entfernt werden soll, so dass der als Ventil ausgestaltete Verschlusskörper 36,36' freigelegt und damit zugreifbar wird und eine Befüllung des Medikamentenbehälters 1 erlaubt.

Für die vorübergehende Anbringung wird der Haltering 70 der Verschluskappe 20 derart auf den Haltekragen 110 der Aufprellkappe 14 aufgesetzt, dass die an seiner Innenseite montierten Riegelzapfen jeweils in eine der zugeordneten Führungskulissen 112 am Haltekragen 110 eingreifen. Anschließend kann durch geeignetes Verdrehen des Halterings 70 gegenüber dem Haltekragen 110 und die entsprechende Führungspaarung in der jeweiligen Führungskulisse 112 die Verschlusskappe 20 derart lösbar am Haltekragen 110 fixiert werden, dass die Siegelmembran 72 an der Oberseite des Verschlussstopfens 12,12' anliegt. Dieser ist somit vor Kontamination durch Partikel, Aerosole und dergleichen selbst im vormontierten Zustand geschützt, so dass insbesondere bei Verwendung einer geeigneten Umverpackung beispielsweise ein Transport des vormontierten System vom Produktionsort an einen Befüllungsort unter durchgängiger Einhaltung steriler Bedingungen möglich ist. Der solchermaßen vormontierte Medikamentenbehälter 1 ist in Fig. 11c gezeigt.

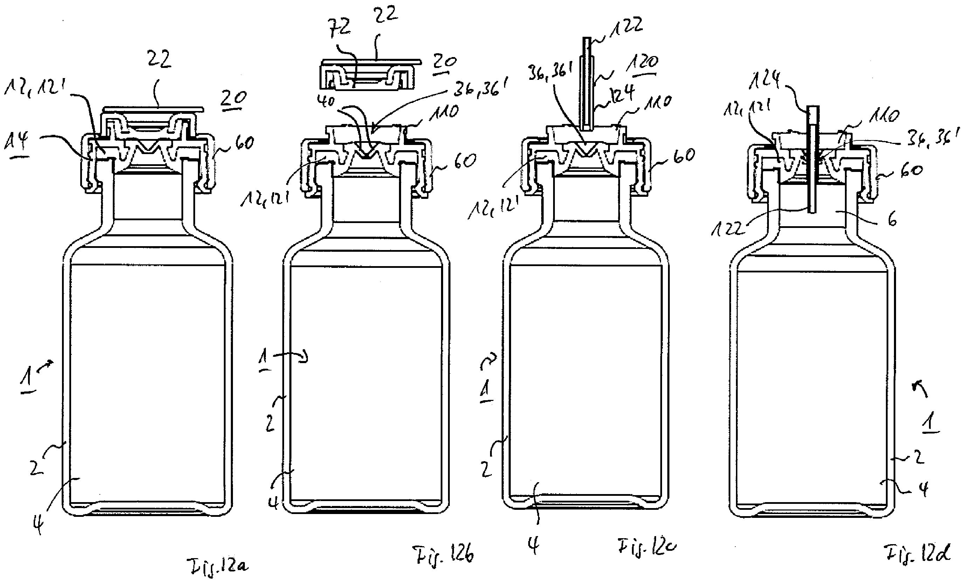

Anschließend kann der vormontierte Medikamentenbehälter 1 in einer Befüllvorrichtung zur Befüllung mit dem Wirkstoff bereitgestellt werden. Gemäß einem Aspekt der vorliegenden Erfindung kann dies aufgrund der Ausgestaltung des Verschlusssystems 10 unter Aufrechterhaltung steriler Bedingungen erfolgen, ohne dass eine erneute Sterilisierung des Medikamentenbehälters 1 am Befüllungsort notwendig wäre. Die Befüllung des Medikamentenbehälters 1 ist in Fig. 12 anhand einer Sequenz von Schritten gezeigt. Als Ausgangspunkt ist dabei in Fig. 12a der vormontierte Behälter 1 nach seiner Verbringung an den Befüllunsgort, aber ansonsten im gleichen Zustand wie in Fig. 11c, dargestellt.

In einem ersten Schritt wird zur Vorbereitung der Befüllung die Verschlusskappe 20 durch "Rückwärtsdrehen", also eine Verdrehung ihres Halterings 70 gegenüber dem Haltekragen 110 in Gegenrichtung zur vorstehend beschriebenen Vormontage, von der Aufprellkappe 14 abgelöst. Dadurch entsteht der in Fig. 12b dargestellte Zustand, bei dem die Siegelmembran 72 vom Verschlussstopfen 12,12' abgehoben wurde, so dass der als Ventil ausgestaltete Verschlusskörper 36,36' freigelegt wird und somit im Zugriff ist. Dies geschieht bevorzugt unter sterilen oder aseptischen Bedingungen innerhalb der Befüllungsanlage.

Der Medikamentenbehälter 1 ist nun bereit zur Befüllung mit Wirkstoff. Dafür wird gemäß einem als eigenständig erfinderisch angesehenen Aspekt innerhalb der nicht dargestellten Befüllvorrichtung ein Füllnadelsystem 120 einer mit einem Vorratsbehälter für die abzufüllende Substanz verbundenen Injektoreinheit an oder bei dem als Ventil ausgeführten Verschlusskörper 36,36' positioniert, wie dies in Fig. 12c gezeigt ist.

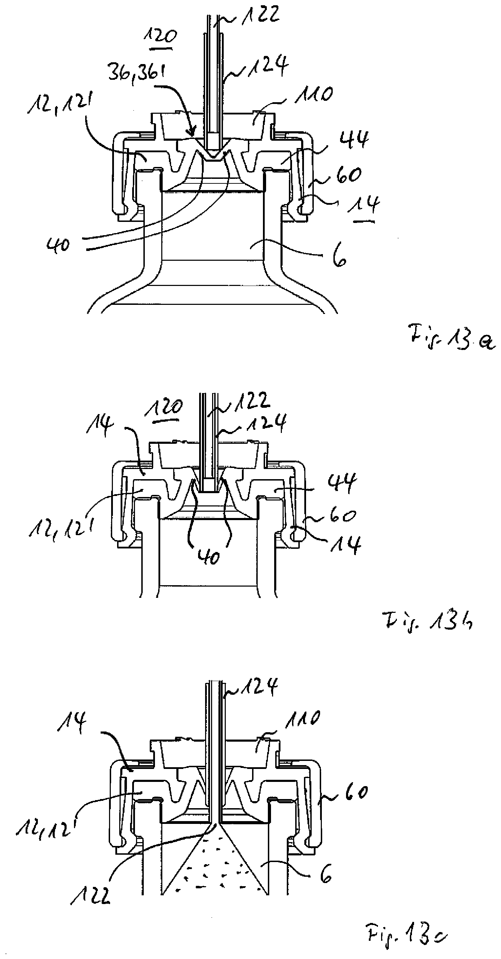

Gemäß einem als eigenständig erfinderisch angesehenen Aspekt umfasst das Füllnadelsystem 120 die eigentliche Füllnadel 122, die in einem äußeren Schutzrohr 124 geführt ist. Zur Befüllung des Medikamentenbehälters 1 mit dem Wirkstoff wird die Füllnadel 122 durch den als Ventil ausgestalteten Verschlusskörper 36,36' hinduch- und in den Behälterinnenraum 4 des Medikamentenbehälters hineingeführt. Zur Verdeutlichung der Wirkungsweise ist in Fig. 13 in vergrößerter Darstellung eine Sequenz dieses Einbringvorgangs dargestellt.

Ausgehend von dem in Fig. 12c gezeigten Zustand wird dabei zunächst das Schutzrohr 124 des Füllnadelsystems 120 mitsamt der darin geführten eigentlichen Injekionsnadel 122 auf den Ventlflanken 40 des im Verschlusskörper 36,36' ausgebildeten Entenschnabelventils aufgesetzt; diese Position ist in Fig. 13a gezeigt. Anschließend wird das Schutzrohr 124 mitsamt darin geführter Füllnadel 122 weiter in den Verschlusskörper 36,36'hineingeschoben, wobei es aufgrund der Ausgesatltung als Entenschnabelventil die Ventilflanken 40 nach außen verdrängt oder verschiebt, ohne dass dabei ein Durchstoßen einer Membran oder dergleichen erforderlich wäre. Beim Einbringen des Schutzrohrs 124 in den Schnabelbereich des Ventils werden somit die das Ventil bildenden, aufeinander zu laufenden Flankenflächen 40 auseinandergeschoben, wobei sich das Ventil öffnet. Dieser Zustand ist in Fig. 13b gezeigt.

Anschließend kann dann die eigentliche Füllnadel 122 innerhalb des Schutzrohrs 124 nach vorn geschoben werden, wobei sie durch den bereits geöffneten Spalt zwischen den Ventilflanken 40 hindurchgeschoben wird. Ein Kontakt der Injetkionsnadel 122 mit freiliegenden Flächen wie beispielsweise den Oberseiten der Ventilflanken 40 kann dabei aufgrund der Verwendung des Schutzrohrs 124 weitgehend vermiedern werden, so dass der Eintrag von Kontaminationen oder Verunreinigungen oder sonstiger Partikel besonders gering gehalten werden kann. Damit erfüllt ein solches Befüllkonzept auch höchste Anforderungen hinsichtlich Aufrechterhaltung von Sterililtät; es ist somit besonders für Befüllprozesse unter aseptischen Bedingungen geeignet. Das System während der Befüllung ist in Fig. 13c und analog auch in Fig. 12d gezeigt.

Beim Herausziehen des Nadelsystems 120 nach dem Einfüllen des Medikaments oder Wirkstoffs schließen sich die Flankenflächen40 des Verschlusskörpers 36,36' dann aufgrund der elastischen Rückstellkräfte im Ventilkörper selbsttätig wieder weitgehend. Damit ist insgesamt gesehen das Risiko eines Eintrags von Verunreinigungen, äußeren Partikeln oder dergleichen besonders gering gehalten, so dass mit einem solchen Verschlusssystem die Befüllung auch unter hohen Reinheitsanforderungen, insbesondere unter Sterilitätsbedingungen, besonders zuverlässig möglich ist.

Nach der Entnahme der Füllnadel 122 aus dem Verschlusskörper 36,36' wird die Verschlusskappe 20 wieder aufgesetzt, und zwar diesmal endgültig, so dass kein erneutes Öffnen des Behälters 1 zerstörungsfrei möglich wäre, so wie dies nach der beschriebenen Vormontage der Fall ist. Die Verriegelung des Halterings 70 der Verschlusskappe 20 auf dem Haltekragen 110 der Aufprellkappe 14 ist dabei analog zur Verriegelung des Sicherungsrings 60 an der Aufprellkappe 14 ausgetaltet. Dazu wird der Haltering 70 relativ zum Haltekragen 110 verdreht, wobei die an der Innenseite seiner zylindrischen Mantelfläche angeordneten Riegelzapfen in der ihnen jeweils zugeordnete Führungskulisse 112 an der Außenseite des Haltekragens 110 geführt werden. Durch dieses Verdrehen wird der jeweilige Riegelzapfen über einen in der jeweiligen Führungskulisse 112 angeordneten Rastzahn mit abgeschrägter Frontfläche hinwegbewegt, was aufgrund der Abschrägung der Frontfläche in dieser Verdrehrichtung möglich ist. Aufgrund der asymmetrischen Kontur des Rastzahns ist ein Rückwärtsdrehen dann aber nicht mehr möglich, so dass der Haltering 70 gegenüber dem Haltekragen 110 der Aufprellkappe 14 dann auch rotationsgesichert ist. Damit ist die Verschlusskappe 20 manipulationssicher auf der Aufprellkappe 14 fixiert, und ein Zugang zum Behälterinnenraum 4 ist nur noch durch Öffnen des Originalitätsverschlusses, also durch Abreißen der Siegelplatte 68 vom Haltering 70 und anschließendes Durchstoßen der Siegelmembran 72 möglich.

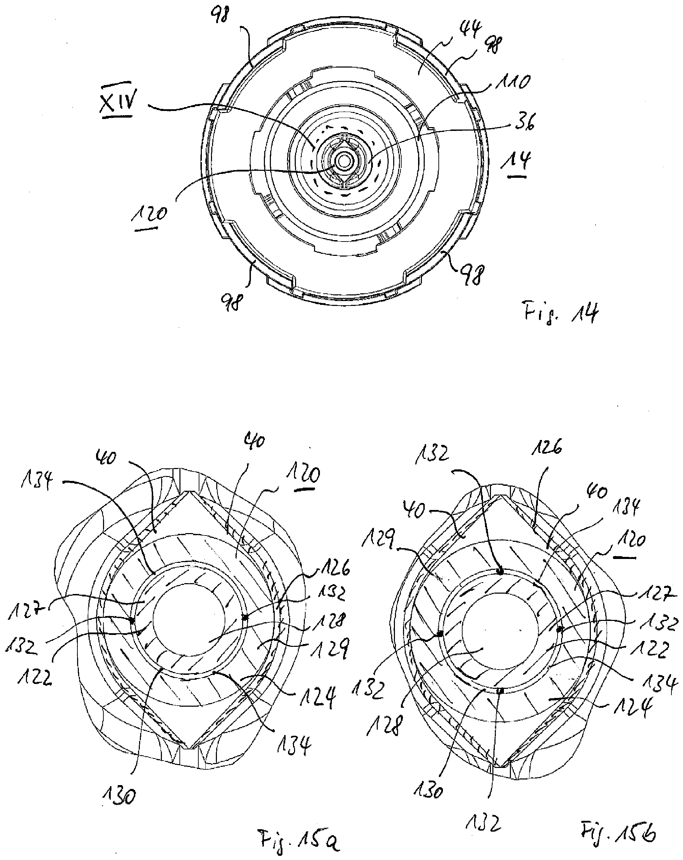

Das Füllnadelsystem 120 bildet somit, wie den genannten Darstellungen in Fig. 13 entnehmbar ist, ein Rohr-in-Rohr-System, mit dem in als eigenständig erfinderisch angesehener Ausgetaltung je nach Bedarf und nach abzufüllendem Wirkstoff unterschiedliche Medienkanäle gebildet werden können. Zur Erläuterung zeigt Fig. 14 die Aufprellkappe 14 mit eingeschobenem Füllnadelsystem 120; Fig. 15 zeigt in zwei unterschiedlichen Varianten den Zentralbereich daraus in vergrößerter Darstellung im Querschnitt. Deutlich erkennbar sind in diesen Darstellungen die bei in das vom Verschlusskörper 36 gebildete Entenschnabelventil eingeschobenem Füllnadelsystem 120 auseinandergespreizten Ventilflanken 40, die in diesem eingeschobenen Zustand eine lippenartige umlaufende Kontur 126 bilden; bei entnommenem Füllnadelsystem 120 schließt sich diese Kontur 126 aufgrund der Rückstellkräfte im elastischen, das Ventil bildenden Material unter Bildung der vorstehend beschriebenen Kontaktlinie 42 wieder.

Wie den vergrößerten Querschnittsdarstellungen in Fig. 15 entnehmbar ist, bildet die Füllnadel 122 einen von ihrem Nadelmantel 127 umschlossenen inneren Medienkanal 128, der zur Nutzung als Medikamentenkanal und somit zum Transport des Medikaments oder Wirkstoffs in den Behälterinnenraum 4 vorgesehen ist. Wie vorstehend beschrieben ist die Füllnadel 122 vom Schutzrohr 124 umgeben. Zwischen dessen Nadelmantel 129 und der Außenseite des Nadelmantels 127 der Füllnadel 122 ist ein Ringspalt 130 ausgebildet. In als eigenständig erfinderisch angesehener Ausgestaltung ist nunmehr vorgesehen, auch diesen Ringspalt 130 als Medienkanal zu nutzen, und zwar insbesondere zum Zu- und Abtransport von Gasen in den bzw. aus dem Behälterinnenraum 4. Einerseits ist es nämlich erforderlich, beim Befüllen des Medikamentenbehälters 1 mit Wirkstoff die im Behälterinnenraum 4 befindliche Luft (oder ein ggf. dort befindliches Schutz- oder Inertgas) entweichen zu lassen; für dieses so genannte "Venting" kann der Ringspalt 130 als Medienkanal genutzt werden. Des Weiteren kann auch, nach erfolgter Einbringung des Wirkstoffs in den Behälterinnenraum 4, die Einbringung von Schutz- oder Inertgas wie beispielsweise Stickstoff in den Behälterinnenraum 4 vorgesehen oder günstig sein. Ein solches Schut- oder Inertgas kann beispielsweise in der Art eines Gaspolsters dazu dienen, einen Kontakt des Wirkstoffs mit Luftsauerstoff und dadurch bedingte chemische Reaktionen zu minimieren oder ganz zu verhindern, und somit die Lagerfähigkeit des Wirkstoffs deutlich zu verbessern.

Der Ringspalt 130 wird somit gemäß dieses Aspekts der Erfindung als Medienkanal für das Venting und/oder zur Einbringung von Schutz- oder anderweitigem Funktionsgas in den Behälterinnenraum 4 genutzt. Dazu ist der Ringspalt mittels einer Anzahl von darin angebrachten Trennwänden 132 in eine Mehrzahl von Spaltsegmenten 134 unterteilt, von denen jedes jeweils für ein zugeordnetes Medium als Medienkanal genutzt werden kann. Art und Anzahl dieser Trennwände 132 und der dadurch gebildeten Spaltsegmente 134 sind dabei bevorzugt im Hinblick auf die Einzelheiten des vorgesehenen Befüllvorgangs, beispielsweise die Anzahl der erforderlichen Ventings und/oder der vorgesehenen Gasbestandteile, geeignet gewählt. Fig. 15a zeigt dabei eine Ausführungsform mit zwei Spaltsegmenten 134 im Ringspalt 130, wohingegen in Fig. 15b eine Variante mit vier Spaltsegmenten 134 gezeigt ist.

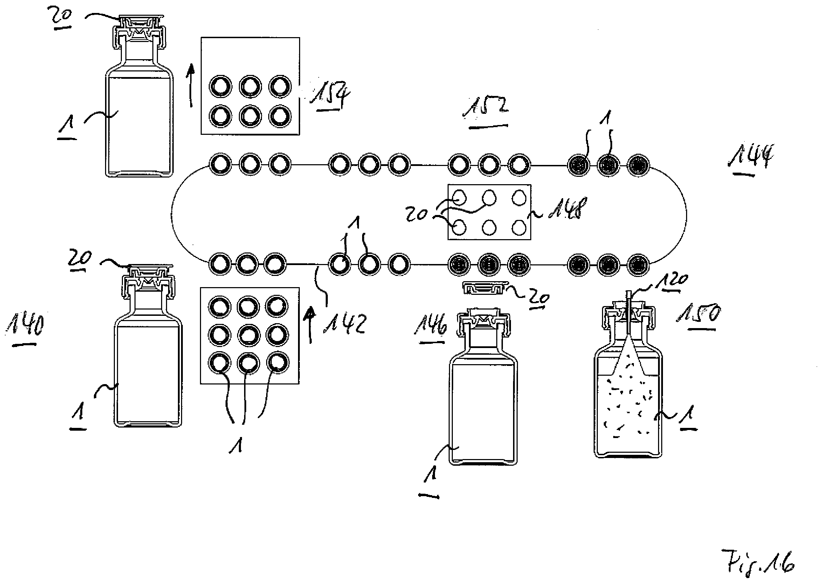

Die gleichzeitige Befüllung einer Mehrzahl solcher Medikamentenbehälter 1 kann automatisiert in einem Befüllprozess durchgeführt werden, wie er schematisch in Fig. 16 gezeigt ist. Neben den jeweiligen schematisch angedeuteten Prozessschritten ist dazu zur Verdeutlichung jeweils einer der Medikamentenbehälter 1 im jeweils aktuellen Zustand gezeigt.

Bei der Befüllung werden zunächst in einem ersten Schritt 140 eine Mehrzahl von Medikamentenbehältern 1 mit vormontiertem Verschlussystem in gemeinsamer Anordnung ("Nesting", im Schema in Draufsicht gezeigt) einem Transportsystem 142 der Befülleinrichtung 144 zugeführt und dort in dem Sinne vereinzelt, dass sie nunmehr im Transportsystem sequentiell hintereinander weiterbefördert werden. Über das Transportsystem 142 gelangen sie sequentiell zu einer Einheit, in der in einem Schritt 146 die Verschlusskappe 20 durch das oben beschrieben "Rückwärtsdrehen" entfernt und einer zwischenzeitlichen Lagerung in einem Zwischenlager 148 zugeführt werden. Anschließend erfolgt in einem Befüllschritt 150 die eigentliche Befüllung nach dem oben beschriebenen Konzept. Schließlich werden die Verschlusskappen 20 aus dem Zwischenlager 148 wieder entnommen und in einem Schritt 152 wieder auf den jeweiligen, nun befüllten Medikamentenbehälter 1 aufgebracht. Die Aufbringung erfolgt dabei in diesem Schritt nunmehr rastend, so dass die jeweilige Verschlusskappe 20 nicht mehr zerstörungsfrei vom jeweiligen Medikamentenbehälter 1 entfernt werden kann. Anschließend werden die nunmehr befüllten und manipulationssicher verschlossenen Medikamentbehälter 1 in einem Schritt 154 wieder bündel- oder palettenartig zu mehreren zusammengefasst und in diesen Einheiten aus der Befüllanlage 144 ausgefördert.

### Bezugszeichenliste

- 1: Medikamentenbehälter
- 2: Behälterwand
- 4: Innenraum
- 6: Behälteröffnung
- 10: Verschlusssystem
- 12: Verschlusstopfen
- 14: Aufprellkappe
- 16: Fixierkappe
- 18: Mündungsöffnung
- 20: Verschlusskappe
- 22: Originalitätsverschluss
- 24: Außenwulst
- 30: Rastelemente
- 32: Öffnung
- 34: Ringdeckel
- 36,36': Verschlusskörper
- 38: Nut
- 40: Ventilflanke
- 42: Kontaktlinie
- 44: Dichtplatte
- 46: Mündungsrand
- 50: Radial-Dichtelement
- 52: Vertiefung
- 54: Verstärkungsring
- 60: Sicherungsring
- 62: Öffnung
- 64: Mantelfläche
- 66: Schnapprippen
- 68: Siegelplatte
- 70: Haltering
- 72: Siegelmembran
- 74: Fixierpunkte
- 80: Mantelfläche
- 82: Führungskulisse
- 84: Mantelfläche
- 86: Axialsegment
- 88, 90: Führungskante
- 92: Rastwulst
- 94: Tangentialsegment
- 96: Führungskante
- 98: zweites Axialsegment
- 100: Übergangsbereich
- 102: Anschlag
- 104: Rastzahn
- 106: Anschlagsfläche
- 110: Haltekragen
- 112: Führungskulisse
- 120: Füllnadelsystem
- 122: Füllnadel
- 124: Schutzrohr
- 126: Kontur
- 127: Nadelmantel
- 128: Medienkanal
- 129: Nadelmantel
- 130: Ringspalt
- 132: Trennwand
- 134: Spaltsegment
- 140: Schritt
- 142: Transportsystem
- 144: Befülleinrichtung
- 146: Schritt
- 148: Zwischenlager
- 150: Befüllschritt
- 152, 154: Schritt

## Patentansprüche

1. Verschlusssystem (10) für einen Medikamentenbehälter (1), dessen Innenraum (4) über eine in der Art einer Flaschenmündung ausgestaltete Behälteröffnung (6) zugänglich ist, umfassend eine Aufprellkappe (14) mit einem eine zentrale Öffnung (32) aufweisenden Ringdeckel (34), an dessen Außenumfang eine Anzahl von mit einer an der Behälteröffnung (6) umlaufend angebrachten Außenwulst (24) in Eingriff bringbaren Rastelementen (30) angeordnet ist, sowie einen einstückigen Verschlussstopfen (12,12') mit einem die zentrale Öffnung (32) des Ringdeckels (34) vollständig ausfüllenden, mit dieser rastend in Eingriff bringbaren Verschlusskörper (36, 36'), **dadurch gekennzeichnet, dass** der Verschlusskörper (36,36') als Entenschnabelventil ausgestaltet ist.

2. Verschlusssystem (10) nach Anspruch 1, bei dem an den Verschlusskörper (36,36') des Verschlusstopfens (12,12') radial umlaufend ein Radial-Dichtelement (50) mit einem an die lichte Weite (I) der Behälteröffnung (6) angepassten, im Hinblick auf die Verformbarkeit des Materials des Verschlussstopfens (12, 12') geringfügig größer als die lichte Weite (I) der Behälteröffnung (6) gewählten Querschnitt angeformt ist.

3. Verschlusssystem (10) nach Anspruch 1 oder 2, weiterhin umfassend einen auf die Aufprellkappe (14) aufschiebbaren Sicherungsring (60), der in einer vollständig auf die Aufprellkappe (14) aufgeschobenen Position mittels einer Anzahl von Schnapprippen (66) an der Aufprellkappe (14) rastbar fixierbar ist.

4. Verschlusssystem (10) nach Anspruch 3, bei dem die oder jede Schnapprippe (66) bei einer Bewegung der inneren Mantelfläche (84) des Sicherungsrings (60) relativ zu einer zu dieser korrespondierenden äußeren Mantelfläche (80) der Aufprellkappe (14) in einer korrespondierenden Führungskulisse (82) geführt ist.

5. Verschlusssystem (10) nach einem der Ansprüche 1 bis 4, an dessen Aufprellkappe (14) ein die zentrale Öffnung (32) umlaufender Haltekragen (110) für eine zugeordnete Verschlusskappe (20) angeformt ist.

6. Verschlusssystem (10) nach Anspruch 5, dessen Verschlusskappe (20) einen Haltering (70) aufweist, an dem eine im montierten Zustand auf dem Verschlusskörper (36,36') aufliegende, durchstechbare Siegelmembran (72) angeordnet ist.

7. Verschlusssystem (10) nach Anspruch 6, an dessen Haltering (70) zur Bildung eines Originalitätsverschlusses (22) eine vom Haltering (70) abreißbare Siegelplatte (68) angeordnet ist.

8. Verschlusssystem (10) nach einem der Ansprüche 5 bis 7, bei dem der Haltekragen (110) der Aufprellkappe (14) außenseitig mit einer Anzahl von Führungskulissen (112) versehen ist, in denen jeweils ein korrespondierender, innenseitig am Haltering (70) der Verschlusskappe (20) angeordneter Riegelzapfen geführt ist.

9. Medikamentenbehälter (1), dessen Innenraum (4) über eine in der Art einer Flaschenmündung ausgestaltete Behälteröffnung (6) zugänglich ist, die mit einem Verschlusssystem (10) nach einem der Ansprüche 1 bis 8 versehen ist.

10. Befüllvorrichtung für einen Medikamentenbehälter (1) nach Anspruch 9, mit einer mit einem Vorratsbehälter für eine abzufüllende Substanz verbundenen Injektoreinheit, die eine in einem äußeren Schutzrohr (124) geführte Füllnadel (122) aufweist.

## Claims

1. Closure system (10) for a medication container (1), whose interior (4) is accessible via a container opening (6) configured in the form of a bottle mouth, comprising a snap-on cap (14) with an annular lid (34) having a central opening (32) and a plurality of latching elements (30) arranged on its outer circumference which can be brought into engagement with an outer bead (24) circumferentially attached to the container opening (6), and a one-piece sealing plug (12, 12') with a closure body (36, 36'), which completely fills the central opening (32) of the annular lid (34) and can be brought into engagement with the same in a snap-in manner, **characterized in that** the closure body (36, 36') is configured as a duckbill valve.

2. Closure system (10) according to claim 1, in which a radial sealing element (50) is integrally formed on the closure body (36, 36') of the closure plug (12, 12') in a radially circumferential manner with a cross section, which is adapted to the clear width (1) of the container opening (6) and selected, with a view to the deformability of the material of the closure plug (12, 12'), to be slightly larger than the clear width (1) of the container opening (6).

3. Closure system (10) according to claim 1 or 2, further comprising a locking ring (60), which can be pushed onto the snap-on cap (14) and which, in a position completely pushed onto the snap-on cap (14), can be fixedly snapped on to the snap-on cap (14) by means of a plurality of snap ribs (66).

4. Closure system (10) according to claim 3, in which the or each snap rib (66) is guided in a corresponding guide slot (82) during a movement of the inner circumferential surface (84) of the locking ring (60) relative to an outer circumferential surface (80) of the snap-on cap (14) corresponding to the same.

5. Closure system (10) according to one of claims 1 to 4, whose snap-on cap (14) has a retaining collar (110), integrally formed thereon and surrounding the central opening (32), for an assigned closure cap (20).

6. Closure system (10) according to claim 5, whose closure cap (20) has a retaining ring (70), on which a puncturable sealing membrane (72) is arranged, which rests on the closure body (36, 36') in the assembled state.

7. Closure system (10) according to claim 6, whose retaining ring (70) has arranged thereon a sealing plate (68) which forms a tamper-evident closure (22) and can be torn off from the retaining ring (70).

8. Closure system (10) according to one of claims 5 to 7, in which the retaining collar (110) of the snap-on cap (14) is provided on the outer side with a plurality of guide slots (112) in which a corresponding lock pin, arranged on the inner side on the retaining ring (70) of the closure cap (20), is respectively guided.

9. Medication container (1), whose interior (4) is accessible via a container opening (6), configured in the form of a bottle mouth, which is provided with a closure system (10) according to one of claims 1 to 8.

10. Filling device for a medication container (1) according to claim 9, with an injector unit, connected to a storage container for a substance to be filled and having a filling needle (122) guided in an outer protection tube (124).

## Revendications

1. Système d'obturation (10) d'un récipient (1) pour médicaments, dont l'espace intérieur (4) est accessible par l'intermédiaire d'un orifice (6) de récipient conçu à la manière d'une embouchure de bouteille, comprenant un capuchon encliquetable (14) pourvu d'un couvercle annulaire (34) comportant un orifice (32) central, sur la circonférence extérieure duquel est placé un nombre d'éléments d'enclenchement (30) susceptibles d'être amenés en engagement avec un bourrelet extérieur (24) monté en périphérie sur l'orifice (6) de récipient, ainsi qu'un bouchon d'obturation (12, 12') en monobloc, pourvu d'un corps d'obturation (36, 36') remplissant totalement l'orifice (32) central du couvercle annulaire (34), susceptible d'être amené en engagement par enclenchement avec celui-ci, **caractérisé en ce que** le corps d'obturation (36, 36') est conçu sous la forme d'une valve en bec de canard.

2. Système d'obturation (10) selon la revendication 1, sur lequel sur le corps d'obturation (36, 36') du bouchon d'obturation (12, 12'), un élément d'étanchéité (50) radial, d'une section transversale adaptée à la largeur intérieure (1) de l'orifice (6) de récipient, sélectionnée de manière légèrement supérieure à la largeur intérieure (1) de l'orifice (6) de récipient au regard de la déformabilité de la matière du bouchon d'obturation (12, 12') est surmoulé de manière périphérique en direction radiale.

3. Système d'obturation (10) selon la revendication 1 ou 2, comprenant par ailleurs une bague de blocage (60) emboîtable sur le capuchon encliquetable (14), qui dans une position totalement emboîtée sur le capuchon encliquetable (14) est susceptible d'être fixée de manière encliquetable au moyen d'un nombre de nervures à déclic (66) sur le capuchon encliquetable (14).

4. Système d'obturation (10) selon la revendication 3, sur lequel, lors d'un déplacement de la surface enveloppante (84) intérieure de la bague de blocage (60) par rapport à une surface enveloppante (80) extérieure lui correspondant du capuchon encliquetable (14), la ou chaque nervure à déclic (66) est guidée dans une coulisse de guidage (82).

5. Système d'obturation (10) selon l'une quelconque des revendications 1 à 4, sur le capuchon encliquetable (14) duquel est surmoulé un collet de maintien (110) périphérique autour de l'orifice (32) central, pour un capuchon d'obturation (20) associé.

6. Système d'obturation (10) selon la revendication 5, dont le capuchon d'obturation (20) comporte une bague de maintien (70) sur laquelle est placée une membrane d'operculage (72) transperçable, reposant sur le corps d'obturation (36, 36') en position montée.

7. Système d'obturation (10) selon la revendication 6, sur la bague de maintien (70) duquel, pour créer une fermeture inviolable (22) est placée une plaque d'operculage (68) arrachable de la bague de maintien (70) .

8. Système d'obturation (10) selon l'une quelconque des revendications 5 à 7, sur lequel le collet de maintien (110) du capuchon encliquetable (14) est muni sur la face extérieure d'un nombre de coulisses de guidage (112), dans lesquelles est guidé chaque fois un tenon de verrouillage correspondant, placé sur la face intérieure de la bague de maintien (70) du capuchon d'obturation (20).

9. Récipient (1) pour médicaments, dont l'espace intérieur (4) est accessible par l'intermédiaire d'un orifice (6) de récipient conçu à la manière d'une embouchure de bouteille, qui est muni d'un système d'obturation (10) selon l'une quelconque des revendications 1 à 8.

10. Dispositif de remplissage d'un récipient (1) pour médicaments selon la revendication 9, pourvue d'une unité d'injection, assemblée avec un récipient de stockage pour une substance à charger, qui comporte une aiguille de remplissage (122) guidée dans un tube protecteur (124) extérieur.
